(19)

Europäisches
Patentamt
European
Patent Office
Office européen
des brevets

(11) **EP 3 050 514 A1**

(12) **EUROPEAN PATENT APPLICATION**

(43) Date of publication:
**03.08.2016 Bulletin 2016/31**

(51) Int Cl.:
*A61B 8/06* (2006.01)    *A61B 8/08* (2006.01)
*A61B 8/00* (2006.01)

(21) Application number: **15172029.9**

(22) Date of filing: **15.06.2015**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **07.01.2015   KR 20150001835**

(71) Applicant: **Samsung Medison Co., Ltd.
Gangwon-do 250-870 (KR)**

(72) Inventor: **Lee, Jae Sung
Incheon (KR)**

(74) Representative: **Frey, Sven Holger
Lorenz & Kollegen
Patentanwälte Partnerschaftsgesellschaft mbB
Alte Ulmer Strasse 2
89522 Heidenheim (DE)**

(54) **ULTRASONIC DIAGNOSTIC APPARATUS AND METHOD FOR CONTROLLING THE SAME**

(57)     The present disclosure provides an ultrasonic diagnostic apparatus, including an ultrasonic probe for transmitting or receiving ultrasound signals; a blood flow detector for collecting blood flow information by detecting blood flows in a subject; a controller for setting at least one focal point in the blood flow of the subject based on the blood flow information; and a beamformer for focusing the ultrasound signals on the at least one focal point.

## FIG.2

**Description**

**BACKGROUND**

1. Field

The present disclosure relates to an ultrasonic diagnostic apparatus and method for controlling the same, which uses echo ultrasound signals reflected off and returning from a subject to image an inside of the subject.

2. Description of the Related Art

Ultrasonic diagnostic apparatuses irradiate ultrasound signals from the surface of a subject toward a target region inside the body, and noninvasively obtains a cross-sectional tomogram of a soft tissue or an image of a blood flow using information about reflected ultrasonic signals (or ultrasonic echo signals).

The ultrasonic diagnostic apparatus is widely used in diagnosing diseases in the heart, abdominal region, urinary organs, and organs unique to women, because it is small, inexpensive, able to display images in real time, and safe without being exposed to radiation like X-rays, as compared to other diagnostic imaging apparatuses, such as X-ray imaging devices, Magnetic Resonance Imaging (MRI) devices, nuclear medicine diagnostic devices, etc.

The ultrasonic diagnostic apparatus may focus ultrasound signals to be transmitted to the subject through beamforming. Beamforming is an important factor that determines resolution, contrast, and signal to noise ratio (SNR) of an ultrasonic image created by the ultrasonic diagnostic apparatus, and thus needs to be appropriately performed to obtain an optimum ultrasound image.

**SUMMARY**

The present disclosure provides an ultrasonic diagnostic apparatus and method for controlling the same, which offers ultrasonic images.

In accordance with an aspect of the present disclosure, an ultrasonic diagnostic apparatus is provided. The ultrasonic diagnostic apparatus includes an ultrasonic probe for transmitting or receiving ultrasound signals; a blood flow detector for collecting blood flow information by detecting blood flows in a subject; a controller for setting at least one focal point in the blood flow of the subject based on the blood flow information; and a beamformer for focusing the ultrasound signals on the at least one focal point.

The blood flow information may include at least one of location of blood flow and distribution of blood flow.

The blood flow detector may use the location and distribution of blood flow detected for each scan line to create a blood flow map.

The controller may set the at least one focal point for each scan line.

The beamformer may form a transmit beam such that the ultrasound signals are focused on the at least one focal point for each scan line.

The beamformer may form a transmit beam per focal point, if there are multiple focal points in the same scan line.

The controller may determine a focal region for each scan line, in which the ultrasound signals are to be focused, based on the at least one focal point.

The beamformer may form a transmit beam such that the ultrasound signals are focused in the focal region determined for each scan line.

The controller may determine a width of the transmit beam to be irradiated to the scan line.

The controller may determine a focusing location and focusing range of the transmit beam such that the width of the transmit beam becomes minimum.

The beamformer may focus ultrasound signals received from the subject in a synthetic aperture beamforming scheme.

The ultrasonic imaging apparatus may further include a memory for storing ultrasound signals received from the subject, wherein ultrasound signals stored in the memory is drawn out and subject to transmit beamforming, such that ultrasound signals received from the subject are focused on the at least one focal point.

The blood flow detector may detect the blood flow location for each scan line of the ultrasound signals, and create the blood flow map for each scan line.

The blood flow detector may detect a blood flow location that changes according to a synthesized signal generated based on the at least one focal point, and update the blood flow map based on the changed blood flow location.

The blood flow detector may include a filtering unit for using a clutter filter to eliminate clutters from a synthesized signal beamformed by the beamformer; a flow detector for converting the synthesized signal with the clutters eliminated into intensity components, and calculating blood flow intensity information for each scan line; a location searching unit for finding the blood flow location for each scan line based on the blood flow intensity information; and a map manager for creating the blood flow map based on blood flow distribution detected for each scan line.

In accordance with another aspect of the present disclosure, a method for controlling an ultrasonic diagnostic apparatus is provided. The method includes performing detection of blood flow by collecting blood flow information by detecting blood flows in a subject; setting at least one focal point in the blood flow of the subject based on the blood flow information; and performing beamforming by focusing the ultrasound signals on the at least one focal point.

Setting at least one focal point in the blood flow of the subject based on the blood flow information may include setting the at least one focal point for each scan line of the ultrasound signals.

Setting at least one focal point in the blood flow of the subject based on the blood flow information may include determining a focal region in which the ultrasound signals are to be focused, if there are multiple focal points in a single scan line, and performing beamforming may include focusing the ultrasound signals in the focal region.

Performing beamforming may include performing transmit beamforming to generate a transmit beam for the ultrasound signals to be focused on the at least one focal point; and performing receive beamforming to focus echo signals reflected from the subject on the at least one focal point.

Performing transmit beamforming may include generating the transmit beam for each focal point if there are multiple focal points in a single scan line.

Determining a focal region in which the ultrasound signals are to be focused may include determining a focusing location and focusing range of the transmit beam such that a width of the transmit beam becomes minimum.

Performing detection of blood flow may include finding the blood flow location for each scan line of the ultrasound signals; and creating the blood flow map for each scan line based on the blood flow location found for each scan line.

Other aspects, advantages, and salient features of the disclosure will become apparent to those skilled in the art from the following detailed description, which, taken in conjunction with the annexed drawings, discloses exemplary embodiments of the disclosure

## BRIEF DESCRIPTION OF THE DRAWINGS

The above and other features and advantages of the present disclosure will become more apparent by describing in detail exemplary embodiments thereof with reference to the attached drawings in which:

FIG. 1 is a perspective view of an ultrasonic diagnostic apparatus, according to an embodiment of the present disclosure;
FIG. 2 is a control block diagram of an ultrasonic diagnostic apparatus, according to an embodiment of the present disclosure;
FIG. 3 illustrates operation of an ultrasonic probe of an ultrasonic diagnostic apparatus, according to an embodiment of the present disclosure;
FIG. 4 is a block diagram of a beamformer of an ultrasonic diagnostic apparatus, according to an embodiment of the present disclosure;
FIG. 5 illustrates transmit beamforming performed by the beamformer of FIG. 4;
FIG. 6 illustrates receive beamforming performed by the beamformer of FIG. 4;
FIG. 7 illustrates a blood flow map created by an ultrasonic diagnostic apparatus, according to an embodiment of the present disclosure;
FIG. 8 is a control block diagram of a blood flow detector, according to an embodiment of the present disclosure;
FIG. 9 illustrates characteristics of a clutter filter;
FIG. 10 illustrates a diagram for explaining detection of blood flow location in scan line a of FIG. 7;
FIG. 11 illustrates a diagram for explaining detection of blood flow location in scan line b of FIG. 7;
FIG. 12 illustrates changes in blood flow over time;
FIG. 13 illustrates beamforming control based on a blood flow map, according to an embodiment of the present disclosure;
FIG. 14 illustrates an updated blood flow map;
FIG. 15A illustrates beamforming control based on a blood flow map, according to another embodiment of the present disclosure;
FIG. 15B illustrates beamforming control based on a blood flow map, according to another embodiment of the present disclosure;
FIG. 16 illustrates beamforming control based on a blood flow map, according to another embodiment of the present disclosure;
FIG. 17 illustrates a diagram for explaining determination of transmit beam width by a controller, according to an embodiment of the present disclosure;
FIG. 18 is a schematic diagram for explaining a synthetic aperture beamforming scheme;

FIG. 19 is a flowchart illustrating a method for controlling an ultrasonic diagnostic apparatus, according to an embodiment of the present disclosure;

FIG. 20 is a flowchart illustrating a method for controlling an ultrasonic diagnostic apparatus, according to another embodiment of the present disclosure; and

FIG. 21 is a flowchart illustrating a method for controlling an ultrasonic diagnostic apparatus, according to another embodiment of the present disclosure.

Throughout the drawings, like reference numerals will be understood to refer to like parts, components, and structures.

## DETAILED DESCRIPTION

Advantages, features, and methods for achieving them will be understood more clearly when the following embodiments are read with reference to the accompanying drawings. The embodiments of the disclosure may, however, be embodied in many different forms and should not be construed as limited to the embodiments set forth herein; rather, these embodiments are provided so that this disclosure will be thorough and complete, and will fully convey the scope of the embodiments of the disclosure to those skilled in the art.

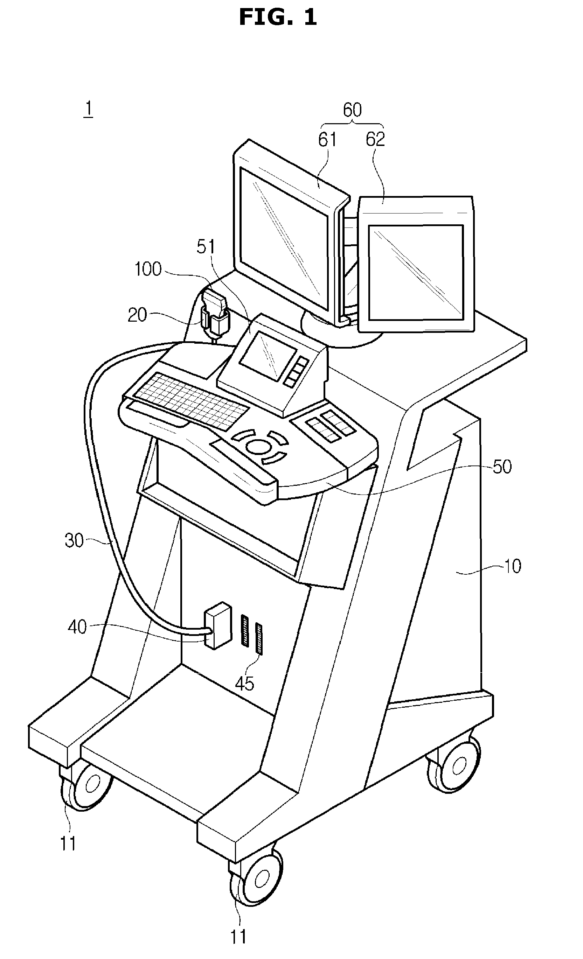

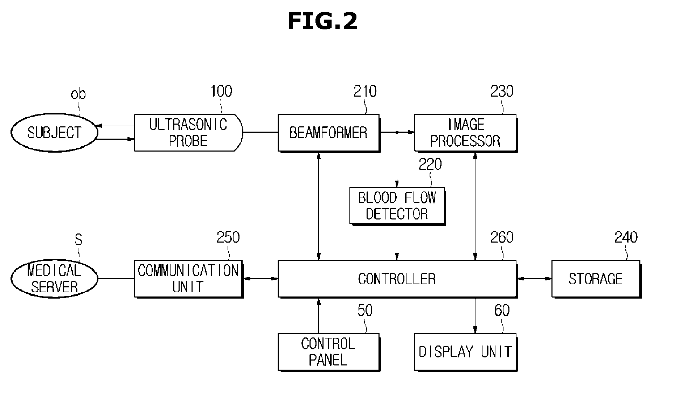

FIG. 1 is a perspective view of an ultrasonic diagnostic apparatus, according to an embodiment of the present disclosure, and FIG. 2 is a control block diagram of an ultrasonic diagnostic apparatus, according to an embodiment of the present disclosure.

Referring to FIGS. 1 and 2, an ultrasonic diagnostic apparatus 1 may include an ultrasonic probe 100, a main unit 10, a control panel 50, and a display unit 60.

The ultrasonic probe 100 is a device that comes in contact with the surface of the subject (ob) for transmitting ultrasound signals or receiving ultrasonic signals reflected from the subject (ob). In other words, the ultrasonic probe 100 may convert ultrasound signals to at least one ultrasound beam and transmit the ultrasound beam to the subject (ob), and receive and output echo ultrasounds reflected from the subject (ob).

Furthermore, the ultrasonic probe 100 may be connected to the main unit 10 of the ultrasonic diagnostic apparatus 1 via a cable 30, for receiving various signals required to control the ultrasonic probe 100, or sending analog or digital signals that correspond to echo ultrasound signals received by the ultrasonic probe 100 to the main unit10.

Although the ultrasonic probe 100 is shown in FIG. 1 to be wiredly connected, it is not limited thereto. For example, the ultrasonic probe 100 may be implemented with a wireless probe to exchange signals with the main unit 10 over a network formed between the ultrasonic probe 100 and the main unit 10.

One or more female connectors 45 may be mounted on the lower front of the main unit 10. The female connector 45 may be mechanically combined with a male connector 40 formed at one end of the cable 30. The cable 30 may connect the ultrasonic probe 100 and the main unit 10.

On the bottom side of the main unit 10, there may be multiple casters 11 for mobility of the ultrasonic apparatus 1. Using the multiple casters 11, the user may fix the ultrasonic diagnostic apparatus 1 at a particular position or move it in a particular direction. Such an ultrasonic diagnostic apparatus 1 may be referred to as a cart-type ultrasonic apparatus.

Alternatively, unlike what is shown in FIG. 1, the ultrasonic diagnostic apparatus 1 may be a portable ultrasonic apparatus that may be carried for long distance. In this case, the portable ultrasonic apparatus may not include the caster 11. For example, the portable ultrasonic apparatus may be a PACS viewer, a smart phone, a laptop computer, a personal digital assistant (PDA), a tablet personal computer (PC), etc., but is not limited thereto.

The main unit 10 may be equipped with the control panel 50. The control panel 50 is a part for receiving commands related to operations of the ultrasound diagnostic apparatus 1. The user may input commands to start examination, select a part to be examined, select an examination type, select a mode for an ultrasound image to be finally output, etc., through the control panel 50. As an example of the mode for the ultrasound image, there may be Amplitude mode (A mode), Brightness mode (B mode), Color Doppler mode (C mode), Doppler mode (D mode), Elastography mode (E mode), Motion mode (M mode), etc.

In an embodiment, the control panel 50 may be located on the top of the main unit 10, as shown in FIG. 1. The control panel 50 may include at least one of switches, keys, a wheel, a joy stick, a trackball, and a knob.

The control panel 50 may further include a sub-display 51. The sub-display 51 may be formed on one side of the control panel 50 for displaying information about manipulation of the ultrasonic diagnostic apparatus 1.

For example, the sub-display 51 may display a menu or guideline required for setting up the ultrasonic diagnostic apparatus 1, or display current the settings of the ultrasonic diagnostic apparatus 1.

The sub-display 51 may be implemented with a touch panel, in which case, the user may input a control command by touching the sub-display 51.

The sub-display 52 may be implemented with e.g., a Liquid Crystal Display (LCD) panel, Light Emitting Diode (LED) panel, an Organic Light Emitting Diode (OLED) panel, or the like.

The main unit 10 may include the display unit 60. The display unit 60 may display ultrasound images obtained in the process of ultrasonic diagnosis. The display unit 60 may be installed in combination with the main unit 10 as shown in FIG. 1, or separately from the main unit 10

There may be one or more probe holders 20 around the control panel 50 to hold the ultrasonic probe 100. Accordingly, the user may keep the probe 200 in the probe holder 20 while the ultrasound diagnostic apparatus 1 is not used.

The display unit 60 may also include multiple display devices 61, 62 to display different images at the same time. For example, the first display device 61 may display a two dimensional (2D) ultrasound image while the second display device 62 displays a three dimensional (3D) image. Alternatively, the first display device 61 may display a B mode image while the second display device 62 displays a D mode image.

Each of the display devices 61, 62 may employ a display means, such as a plasma display panel (PDP), a liquid crystal display (LCD) panel, a light emitting diode (LED) panel, an organic LED (OLED) panel, an active-matrix OLED (AMOLED) panel, etc., or a sound output means, such as a speaker, but is not limited thereto.

Furthermore, as shown in FIG. 2, the ultrasonic diagnostic apparatus 1 may include a beamformer 210 for performing beamforming, a blood flow detector 220 for collecting blood flow information, an image processor 230 for creating ultrasound images, a storage 240 for storing data, a communication unit 250 for communicating with external devices, and a controller 260 for controlling the respective components. Details of the components of the ultrasonic diagnostic apparatus 1 in accordance with an embodiment will now be described.

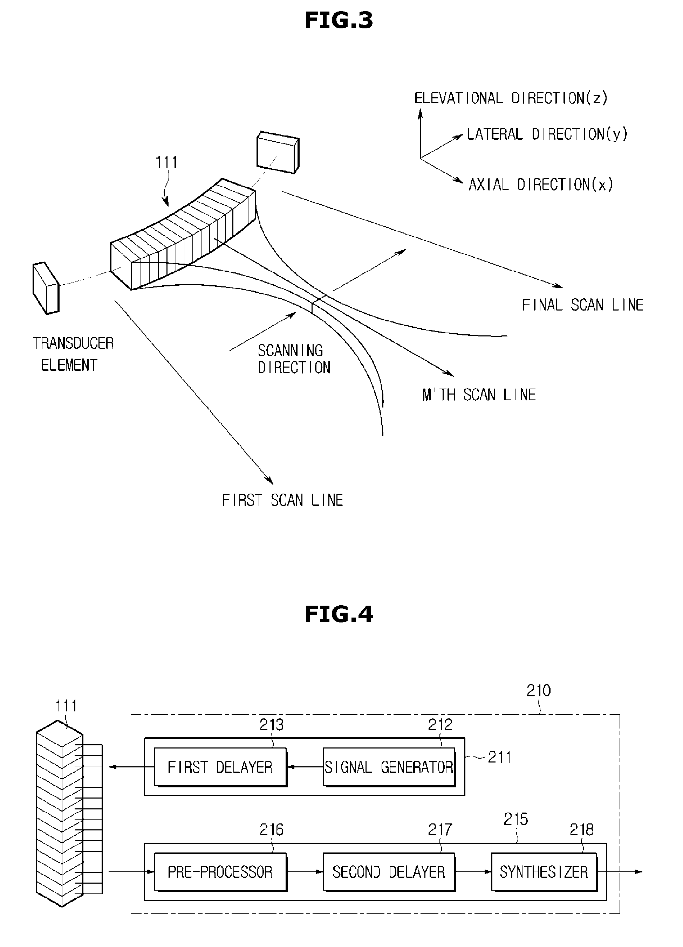

FIG. 3 illustrates operation of an ultrasonic probe of an ultrasonic diagnostic apparatus, according to an embodiment of the present disclosure.

The ultrasonic probe 100 may include a transducer array 111 comprised of a plurality of transducer elements, as shown in FIG. 3.

The transducer elements may convert ultrasound signals to electric signals, and vice versa. For this, the transducer elements may be implemented with magnetostrictive ultrasonic transducers that use magnetostrictive effects of a magnetic substance, piezoelectric ultrasonic transducers that use piezoelectric effects of a piezoelectric material, piezoelectric micromachined ultrasonic transducers (pMUTs), or the like, or may also be implemented with capacitive micromachined ultrasonic transducers (cMUTs) that transmit/receive ultrasounds using vibration of hundreds or thousands of micromachined thin films.

The transducer array 111 may have the transducer elements arranged linearly, or on a convex plane as shown in FIG. 3. The transducer array 111 may be implemented to be multidimensional, e.g., M*N.

As shown in FIG. 3, a 3D space subject to ultrasonic imaging may be defined by the X-axis corresponding to axial direction, the Y-axis corresponding to lateral direction, and the Z-axis corresponding to elevational direction.

The ultrasonic diagnostic apparatus 1 may form a plurality of scan lines along the axial direction to transmit or receive ultrasound signals, and create 2D ultrasound images based on ultrasound signals received from the subject.

Spatial resolution of a two dimensional (2D) ultrasound image may be determined by axial and lateral resolution. Axial resolution refers to the ability to distinguish two objects that lie along the axis of the ultrasound beam, and the lateral resolution refers to an ability to distinguish two objects that lie on a line perpendicular to the axis of the ultrasound beam.

Axial resolution is determined by the pulse width of the transmit ultrasound signal, and a higher frequency ultrasound signal with a shorter pulse width yields better axial resolution.

Since the lateral resolution and elevational resolution are determined by the width of the ultrasound beam, the narrower the width of the ultrasound beam, the better the lateral resolution. To improve the resolution of an ultrasound image, the lateral resolution in particular, the beamformer 210 may focus ultrasound signals transmitted from a plurality of transducer elements to a focal point on a scan line, thereby increasing the resolution around the focal point. Alternatively, it may increase overall image resolution by focusing signals on several focusing points on a single scan line while absorbing a damage that slows down the frame rate. The beamformer 210 may form a receive ultrasound beam with a narrow width by focusing ultrasound signals on every imaging point for imaging. As such, forming a transmit or receive ultrasound beam to focus ultrasound signals is referred to as beamforming.

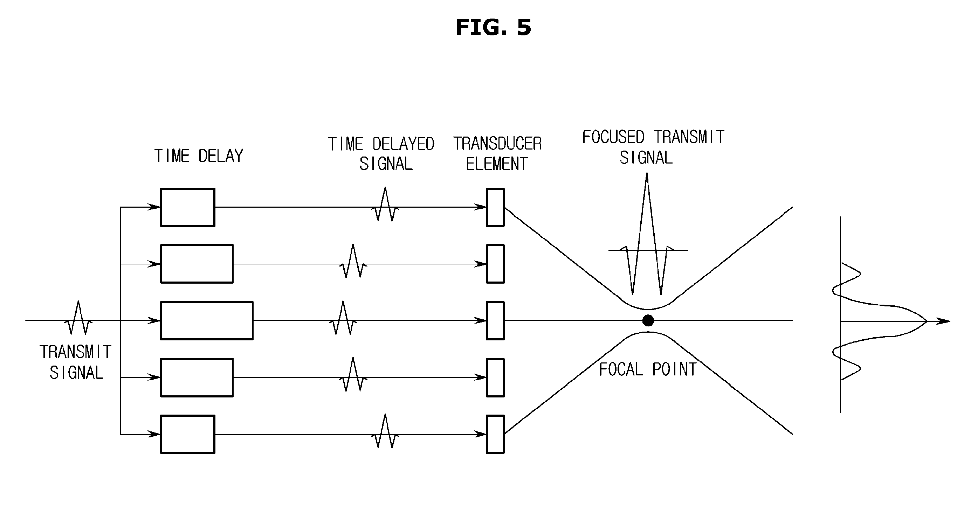

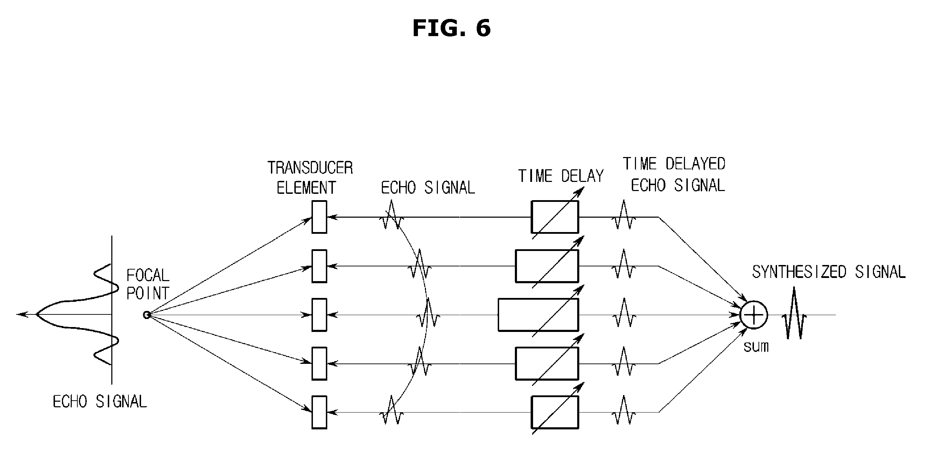

FIG. 4 is a block diagram of a beamformer of an ultrasonic diagnostic apparatus, according to an embodiment of the present disclosure. FIG. 5 illustrates transmit beamforming by the beamformer of FIG. 4, and FIG. 6 illustrates receive beamforming by the beamformer of FIG. 4.

The beamformer 210 performs beamforming. Beamforming may be performed for each scan line. As shown in FIG. 3, to obtain a 2D cross-sectional ultrasound image, ultrasound signals need to be transmitted or received in at least one scan line, and thus beamforming may be performed for each scan line.

Specifically, the beamformer 210 may include a transmitter 211 for performing transmit beamforming that focuses ultrasounds to be transmitted to the subject ob, and a receiver 215 for performing receive beamforming that focuses ultrasounds reflected from the subject ob.

The transmitter 211 may form at least one transmit beam to be irradiated to the subject ob through transmit beamforming. Specifically, the transmitter 211 may include a signal generator 212, and a first delayer 213.

The signal generator 212 may generate a transmit signal, which is an ultrasound signal to be transmitted to the subject ob. As ultrasounds to be generated by the transducer array 111 are determined depending on the transmit signal generated by the signal generator 212, the transmit signal may be determined based on operating environments of the ultrasonic diagnostic apparatus 1, such as properties of the subject ob, a part to be examined, purpose of examination, or the like. The transmit signals have a form of pulses. That is, the signal generator 212 may generate pulses with a proper frequency and phase according to the operating environments of the ultrasonic diagnostic apparatus 1.

The first delayer 213 may apply time delays for the transmit signal generated by the signal generator 212 to be focused on a focal point. In other words, the first delayer 213 may control output timing of the transmit signal so that a transmit beam focused on a focal point may be formed. Forming a transmit beam will be described in detail in connection with FIG. 5.

As shown in FIG. 5, the distances between respective transducer elements and a focal point may be different. Accordingly, the first delayer 213 may apply a proper time delay to a transmit signal to be input to each transducer element such that ultrasounds transmitted from respective transducer elements may arrive at a focal point at the same time. Specifically, the first delayer 213 may apply the transmit signal late to a transducer element located near the focal point, while applying the transmit signal early to a transducer element located far from the focal point.

With such time delaying, ultrasounds generated according to the transmit signals may simultaneously arrive at the focal point. That is, with the time delay applied by the first delayer 213, a transmit beam focused on the focal point may be formed.

In the meantime, as shown in FIG. 3, since a plurality of scan lines are required to obtain a 2D ultrasound image, the transmitter 211 may form a transmit beam per scan line.

The transducer array 111 may irradiated the transmit beam to the subject ob, and receive echo ultrasounds returning from the subject ob. The echo ultrasounds received may be converted by the respective transducer elements to electrical ultrasound signals. Such ultrasound signals converted by the transducer elements may be referred to as echo signals.

Turning back to FIG. 4, the receiver 215 may perform receive beamforming to output a synthetic signal. Receive beamforming refers to superposition of echo signals output from the transducer array 111 at a focal point. Specifically, the receiver 215 may include a pre-processor 216, a transmit delayer 217, and a synthesizer 218.

The pre-processor 216 may pre-process echo signals. The pre-processor 216 may amplify the echo signal to adjust a gain of the echo signal or compensate for attenuation with respect to the depth of the subject ob. For this, the pre-processor 216 may include a variable gain amplifier (VGA) for controlling the gain of an input signal.

Furthermore, the pre-processor 216 may cancel noise of the echo signal. For this, the pre-processor 216 may include a low pass filter for canceling noise of the echo signal.

The transmit delayer 217 may apply predetermined time delays to a plurality of echo signals to compensate for time differences between the echo signals, and synthesize the plurality of echo signals with time differences compensated. Time delay and synthesis will now be described in detail in connection with FIG. 6.

Referring to FIG. 6, the ultrasounds reflected from the subject ob may go back to the transducer array 111. Like the occasion where ultrasounds are transmitted to a focal point, arrival times of echo ultrasounds returning from the focal point may also be different depending on differences in distance between the focal point and the respective transducer elements. The transmit delayer 217 may then compensate for the time differences between the plurality of echo signals by applying appropriate delay times to the echo signals output from the transducer elements at different times. Specifically, the transmit delayer 217 may have an echo signal from a transducer element near the focal point delayed long while having an echo signal from a transducer far from the focal point delayed short, thereby compensating time differences between the plurality of echo signals.

The synthesizer 218 may synthesize the plurality of echo signals with the time differences compensated. The synthesizer 218 may apply respective weights to the echo signals and synthesize the weighted echo signals, thereby relatively emphasizing or attenuating a particular echo signal. The weights may be determined in an independent beamforming scheme, e.g., data-independent beamforming, or fixed beamforming, or an adaptive beamforming scheme, e.g., data-dependant beamforming, or adaptive beamforming.

The synthesizer 218 may synthesize the plurality of echo signals and output the resultant synthesized signal. The synthesizer 218 may demodulate and output the synthesized signal. Specifically, the synthesizer 218 may separate and output the synthesized signal into in-phase component signals (I signals) and quadrature component signals (Q signals).

In an embodiment, the beamformer 210 may output multiple synthesized signals by performing receive and transmit beamforming for each scan line. In this regard, focal points on which ultrasounds are to be focused may be set differently for respective scan lines. A focal point of each scan line may be determined based on blood flow information. Details of the blood flow information will be described below.

Turning back to FIG. 2, the image processor 230 may create an ultrasound image based on a synthesized signal output from the beamformer 210. For example, the image processor 230 may create at least one of A mode images, B mode images, C mode images, D mode images, E mode images, and M mode images, based on the synthesized signal.

The image processor 230 may be implemented in software, or with one or more processors. For example, the image processor 230 may be implemented with a universal Graphic Processing Unit (GPU). Although the image processor 230 is shown to be separated from the controller 260 in FIG. 2, the image processor 230 and the controller 260 may be implemented in a single unit.

A storage 240 may store various data required for operation of the ultrasonic diagnostic apparatus 1. For example, the storage 240 may store an operating system of the ultrasonic diagnostic apparatus 1 or many different applications that may run in the ultrasonic diagnostic apparatus 1. Furthermore, the storage 240 may store temporary data produced in operation of the ultrasonic diagnostic apparatus 1. For example, the storage 240 may store ultrasound images created by the image processor 230, or synthesized signals output from the beamformer 210. The storage 240 may also store blood flow information collected by the blood flow detector 220, or a blood flow map generated based on the blood flow information.

The storage 240 may include e.g., a high-speed random access memory (RAM), a magnetic disk, a static RAM, a dynamic RAM, a read only memory (ROM), etc., but is not limited thereto. Furthermore, the storage 240 may be implemented in a form detachable from the ultrasonic diagnostic apparatus 1. For example, the storage 240 may include a compact flash (CF) card, a secure digital (SD) card, a smart media (SM) card, a multimedia card (MMC), or a memory stick, but is not limited thereto.

The communication unit 250 may be connected to another device for exchanging data with the other device. Especially, the communication unit 250 may be connected to another device over a network for exchanging various data required for image matching. For example, the communication unit 250 may be connected to a medical server S that stores medical records of the subject ob and receive medical information, such as a medical history, treatment schedule, etc., for the subject ob, from the medical server S. The communication unit 250 may also send an ultrasound image created by the image processor 230 to the medical server S.

The communication unit 250 may communicate data with other devices according to various wired/wireless communication protocols, but preferably based on a Digital Imaging and Communications in Medicine (DICOM) standard.

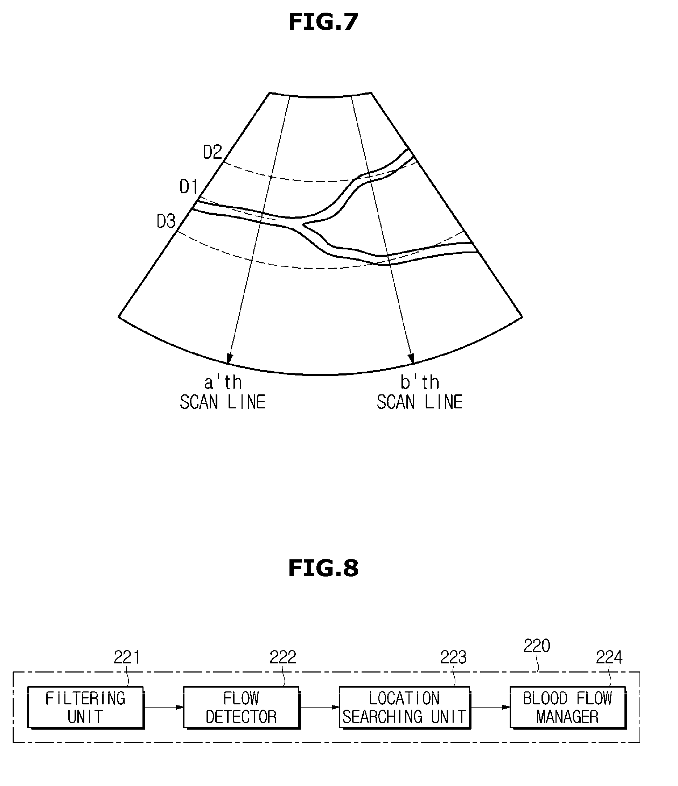

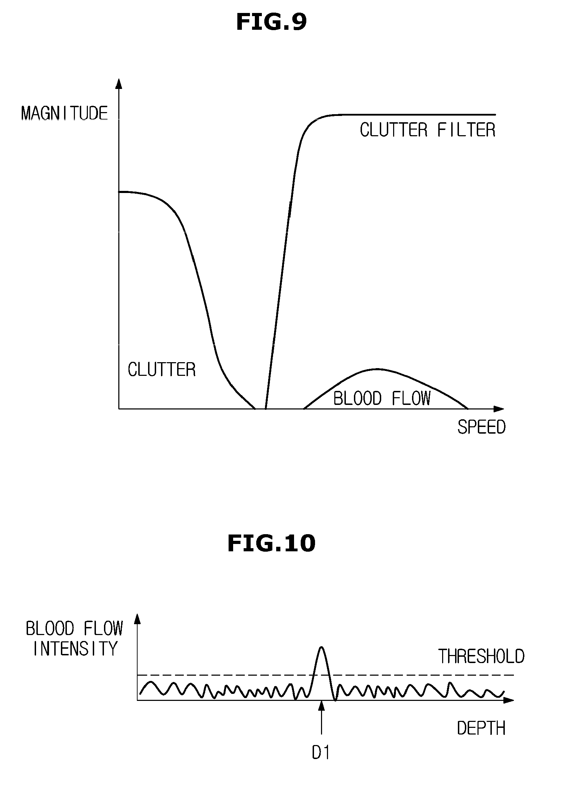

FIG. 7 illustrates a blood flow map created by an ultrasonic diagnostic apparatus, according to an embodiment of the present disclosure, FIG. 8 is a control block diagram of a blood flow detector, according to an embodiment of the present disclosure, and FIG. 9 illustrates characteristics of a clutter filter.

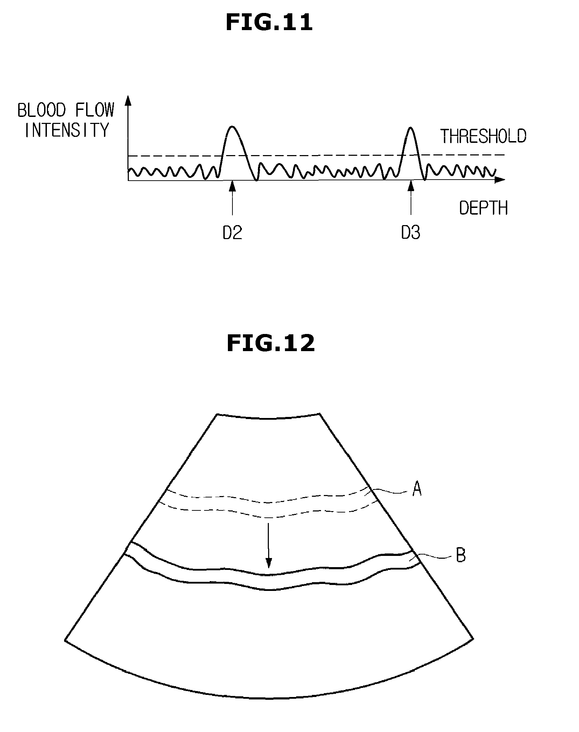

FIG. 10 illustrates a diagram for explaining detection of blood flow location in scan line a of FIG. 7, and FIG. 11 illustrates a diagram for explaining detection of blood flow location in scan line b of FIG. 7.

The blood flow detector 220 may collect blood flow information by detecting blood flows in the subject ob. The blood flow information refers to information about location or distribution of blood flow, that may be obtained for each scan line.

The blood flow detector 220 may create a blood flow map based on the blood flow information, and manage the blood flow map. That is, the blood flow map may be created based on information about location or distribution of blood flow. The ultrasonic diagnostic apparatus 1 may perform beamforming based on the blood flow map.

Specifically, the blood flow map may have a structure corresponding to the ultrasound image created by the ultrasonic diagnostic apparatus 1. For example, if the transducer array 111 of the ultrasonic diagnostic probe 100 has a convex form, an ultrasound image may be created to have a sector shape and even the blood flow map may also be created in the same sector shape as that of the ultrasound image. An embodiment of the blood flow detector 220 will now be described.

Referring to FIGS. 7 to 9, the blood flow detector 220 may include a filtering unit 221, a flow detector 222, a location searching unit 223, and a blood flow map manager 224.

The filtering unit 221 may eliminate clutter signals. Even though the beamformer 210 focuses ultrasound signals on the blood flow, the synthesized signal output from the beamformer 210 may include Doppler signals reflected off from the blood flow and clutter signals reflected off from tissues other than the blood flow. The filtering unit 221 may thus use a predetermined clutter filter to eliminate clutter signals that correspond to noise.

The clutter filter may be implemented with a high pass filter that filters out clutter signal components below a predetermined level in movement but only passes Doppler signals large in movement that corresponds to the blood flow. However, the clutter filter is not limited thereto. The clutter filter may be implemented even with an adaptive filter that adaptively selects an optimum cut-off for an input signal.

Accordingly, the filtering unit 221 may only output Doppler signals by excluding clutter signals from the synthesized signal output from the beamformer 210. The Doppler signals may include I signals and Q signals.

The flow detector 222 may convert the Doppler signals output from the filtering unit 221 into intensity components, to calculate blood flow intensity information. The blood flow intensity information may be calculated for each scan line. As described above, as the beamformer 210 may output synthesized signals along the plurality of scan lines, the flow detector 222 may also calculate blood flow intensity information for each scan line.

The blood flow intensity may be calculated based on absolute values of I signals and Q signals. That is, it may be calculated based on the following equation 1:

$$Y(s,z) = \left\{ \sum_{l=1}^{L} \sqrt{I_l(s,z)^2 + Q_l(s,z)^2} \right\}$$

(1)

where $I_l$ refers to an ensemble number of the Doppler signal, s refers to a number of a scan line, and z refers to a depth of the scan line. The blood flow intensity Y may thus be calculated based on absolute values of the I and Q signals of the Doppler signal.

Specifically, $Y(s, z)$ refers to a blood flow intensity that corresponds to the depth z of the s'th scan line, $I_l(s,z)$ refers to an I signal that corresponds to the depth z of the s'th scan line of the Doppler signal with the $I_l$ ensemble number, and $Q_l(s,z)$ refers to a Q signal that corresponds to the depth z of the s'th scan line of the Doppler signal with the $I_l$ ensemble number.

As such, the blood flow intensity information may be generated depending on depth of the scan line. Specifically, the a'th scan line of FIG. 7 has blood flow intensity information as shown in FIG. 10, and the b'th scan line of FIG. 7 has blood flow intensity information as shown in FIG. 11.

The location searching unit 223 may find location and distribution of blood flow based on the blood flow intensity information. Specifically, the location searching unit 223 may search for where the blood flow intensity is above a threshold, thereby finding location of blood flow for each scan line. Information about distribution of blood flow may be obtained based on the location of blood flow found per scan line. The threshold may be predetermined or determined by the user. Alternatively, the threshold may be dynamically calculated based on the distribution of blood flow intensity information.

As shown in FIGS. 10 and 11, the location searching unit 223 may find depth D1 where the blood flow intensity appears to be greater than the threshold in the scan line a, as a location of blood flow, and find depths D2 and D3 where the blood flow intensity appears to be greater than the threshold in the scan line b, as locations of blood flow. As such, information about distribution of entire blood flows may be obtained by finding locations of blood flow for respective scan lines.

The blood map manager may create a blood flow map based on the location and distribution of blood flow found by the location searching unit 223. Specifically, the blood flow map manager may create a blood flow map based on the location and distribution of blood flow detected for each scan line as shown in FIG. 7. As the location of blood flow keep changing due to to the movement of the ultrasonic probe 100 or the movement of the subject ob, the blood flow detector 220 may keep updating the blood flow map in the aforementioned method for creating the blood flow map.

However, the method for creating the blood flow map is not limited thereto. The blood flow detector 220 may create a blood flow map based on various methods of detecting flows of blood. For example, the blood flow detector 220 may create a blood flow map based on location of blood flow found in a space domain method, a spectral domain method, image comparison method, etc.

The space domain method is a method for detecting movement of location of blood flow by analyzing spatial correlation of movement of a region established as blood flow in an ultrasound image; the spectral domain method is a method for detecting location of blood flow by applying a matching filter to a signal in a spectral domain; and the image comparison method is a method for detecting location of blood flow based on changes in ultrasound images.

Turning back to FIG. 2, the controller 260 may control overall operation of respective components of the ultrasonic diagnostic apparatus 1. The controller 260 may correspond to one or more processors. The processor may be implemented in an array of multiple logic gates, or in a combination of a universal microprocessor and a memory that stores a program executable in the microprocessor.

The controller 260 may control the beamformer 210 and image processor 230 to create an ultrasound image, and control the display unit 60 to display the ultrasound image. The controller 260 may also control the blood flow detector 220 to have the blood flow map represent the location and distribution of blood flow in the subject ob in real time. Although the blood flow detector 220 and the controller 260 are shown to be separate from each other in FIG. 2, the blood flow detector 220 may be incorporated in the controller 260 or the beamformer 210.

Especially, the controller 260 may control the beamformer 210 to focus the ultrasound signal on the blood flow, based on the blood flow map created in real time. Beamforming control of the controller 260 will now be described in detail.

FIG. 12 illustrates changes in blood flow over time.

As shown in FIG. 12, as the relative location of the ultrasonic probe 100 and the blood vessel changes due to movement of the ultrasonic probe 100 or subject ob, location and distribution of blood flow may keep changing while an ultrasound image is obtained. For example, blood flow of location A may be shifted to location B due to a movement of the ultrasonic probe 100 or subject ob.

Thus, to indicate a blood flow more clearly in the ultrasound image, the beamformer 210 needs to be controlled to focus the ultrasound signal to a changed location of blood flow. For this, the controller 260 may determine a focal point on which ultrasound signals are to be focused, based on the blood flow map, and the beamformer 210 may perform beamforming for the ultrasound signals to be focused on the focal point determined by the controller 260. Details of beamforming control based on the blood flow map will now be described with specific embodiments.

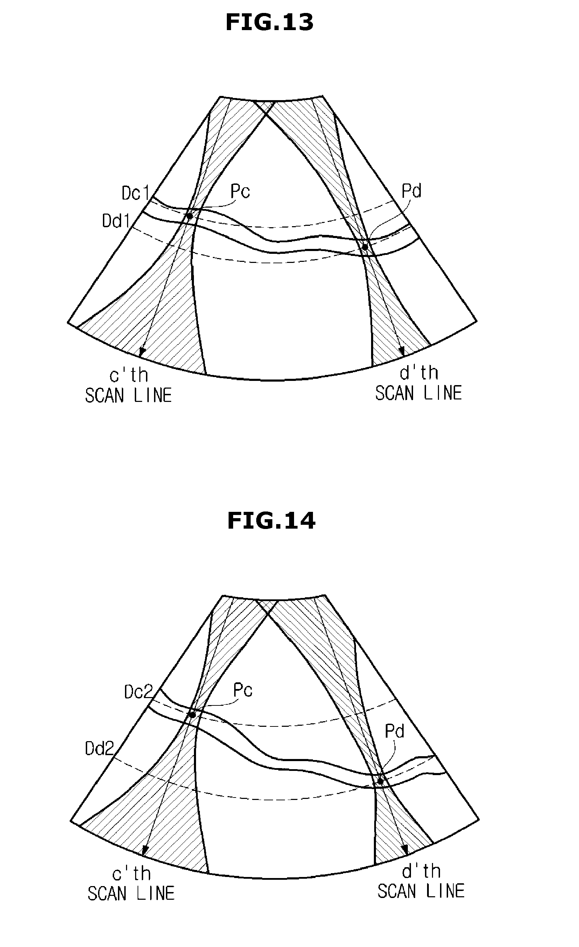

FIG. 13 illustrates beamforming control based on a blood flow map, according to an embodiment of the present disclosure, and FIG. 14 illustrates an updated blood flow map.

The controller 260 may determine a focal point based on a blood flow map created by the blood flow detector 220. Specifically, the controller 260 may determine a focal point per scan line to correspond to a blood flow location that appears on the blood flow map.

As shown in FIG. 13, the controller 260 may determine focal point Pc of the c'th scan line to have depth Dc 1 in the blood flow, and focal point Pd of the d'th scan line to have depth Dd1 in the blood flow.

The beamformer 210 may perform transmit and receive beamforming on the ultrasound signals for the focal points determined by the controller 260. Specifically, the beamformer 210 may generate a transmit beam per scan line in order for the ultrasound signal to be focused on the focal point in the aforementioned transmit beamforming scheme. In other words, the beamformer 210 may generate a transmit beam by focusing the ultrasound signal on the focal point Pc in the c'th scan line, and generate a transmit beam by focusing the ultrasound signal on the focal point Pd in the d'th scan line.

In the meantime, as described above, as the location of blood vessel keeps changing in capturing ultrasound images, if the beamformer 210 outputs a synthesized signal through receive beamforming, the blood flow detector 220 may update the blood flow map as shown in FIG. 14, based on the synthesized signal.

The controller 260 may re-determine a focal point based on the updated blood flow map. For example, the controller 260 may determine the focal point Pc of the c'th scan line to have depth Dc2 in the blood flow and focal point Pd of the d'th scan line to have depth Dd2 in the blood flow, based on the updated blood flow map.

Once focal points are re-determined by the controller 260, the beamformer 210 may generate transmit beams with respect to the re-determined focal points.

As such, an ultrasound image that contains a clearer blood flow image may be provided by dynamically updating the blood flow map and dynamically changing focal points based on the updated blood flow map. This may lead to facilitation of examination of blood vessels of the user.

In the meantime, the controller 260 may display the change of focal points in the ultrasound image as the focal points change as described above.

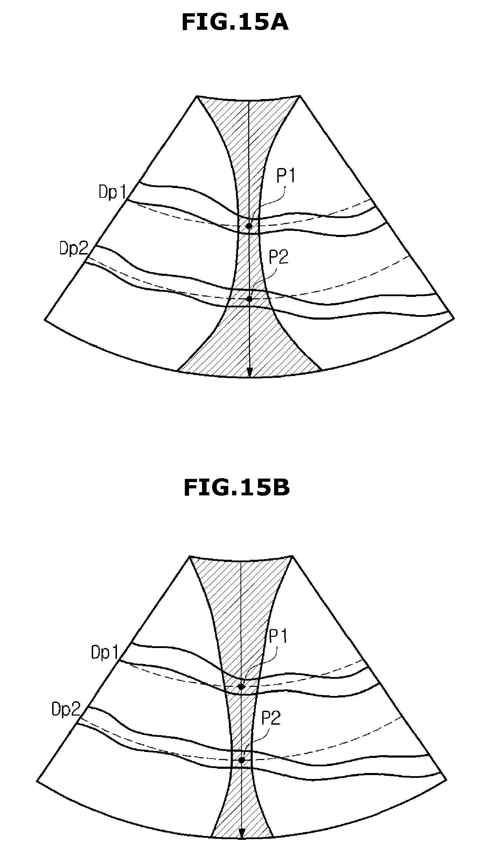

FIG. 15A illustrates beamforming control based on a blood flow map, according to another embodiment of the present disclosure, and FIG. 15B illustrates beamforming control based on a blood flow map, according to another embodiment of the present disclosure.

As shown in FIGS. 15A and 15B, multiple blood flows may be located in a single scan line. In this case, the controller 260 may set multiple focal points in the single scan line.

With the multiple focal points for the single scan line, the beamformer 210 may generate multiple transmit beams for the single scan line. The beamformer 210 may generate a first transmit beam by focusing ultrasound signals on a focal point P1 set at depth Dp1 as shown in FIG. 15A, and generate a second transmit beam by focusing ultrasound signals on a focal point P2 set at depth Dp2 as shown in FIG. 15B.

The beamformer 210 may output a first synthesized signal by beamforming a first echo signal that corresponds to the first transmit beam, and output a second synthesized signal by beamforming a second echo signal that corresponds to the second transmit beam. The image generator may create an ultrasound image for the scan line, based on the first and second echo signals. For example, the image generator may create a part adjacent to the first focal point P1 based on the first synthesized signal, and create a part adjacent to the second focal point P2 based on the second synthesized signal.

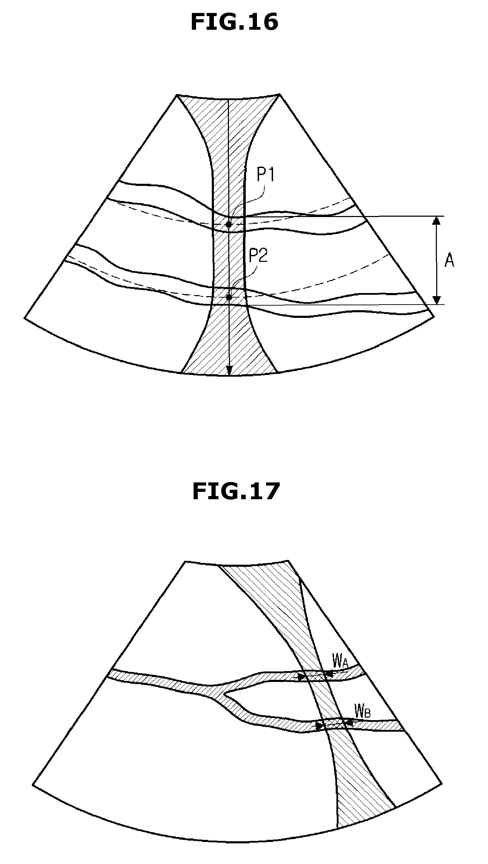

FIG. 16 illustrates beamforming control based on a blood flow map, according to another embodiment of the present disclosure.

Although it has been described that the controller 260 determines focal points based on the blood flow map, the controller 260 may determine a focal region into which ultrasounds are to be focused based on focal points.

As shown in FIG. 16, the controller 260 may set multiple focal points P1, P2 based on a blood flow map. The controller 260 may then set a focal region A based on the multiple focal points P1, P2.

The focal region A may be set to have all the multiple focal points P1, P2. Although the focal region A is shown to be set only by taking into account focal points P1, P2 in a single scan line, the controller 260 may set the focal region by using focal points in different scan lines.

The beamformer 210 may generate a transmit beam such that ultrasound signals may be focused in the focal region A set by the controller 260. The ultrasonic diagnostic apparatus 1 may increase a beamforming rate because it sets the focal region A for performing beamforming.

FIG. 17 illustrates a diagram for explaining determination of transmit beam width by the controller 260, according to an embodiment of the present disclosure.

The controller 260 may determine a width of transmit beam for each scan line. As described above, the narrower the width of transmit beam, the higher the resolution of the ultrasound image. As the width of transmit beam is determined based on a focal distance and aperture size, the controller 260 may determine the focal distance and aperture size such that a sum of transmit beam widths in the location and distribution of blood flow for each scan line is minimized.

Specifically, the controller 260 may determine a width of transmit beam based on the following equation 2:

$$W = W_1 + W_2 + \cdots W_n \tag{2}$$

where, transmit beam width W of a scan line is a sum of transmit beam widths $W_1$ to $W_n$ at multiple blood flow locations (1 to n) in the same scan line, referring to a width of transmit beam transmitted along the scan line. A with of transmit beam at the k'th blood flow location is calculated in the following equation 3:

$$\begin{cases} W_k = \dfrac{1.2 \cdot \lambda}{D} \cdot z_k, & \left( z_k \geq \dfrac{1.2\lambda \cdot Z_F \cdot D}{1.2\lambda \cdot Z_F + D^2} \right) \\ W_k = D\left(1 - \dfrac{z_k}{Z_F}\right), & \left( z_k < \dfrac{1.2\lambda \cdot Z_F \cdot D}{1.2\lambda \cdot Z_F + D^2} \right) \end{cases} \tag{3}$$

where, $Z_k$ refers to a location of the depth of the k'th blood flow, $\lambda$ refers to a wavelength, D refers to an aperture size, and $Z_F$ refers to a focal distance. In equation 3, $Z_k$ and $\lambda$, are determined in advance, and the transmit beam width at the k'th blood flow location is determined based on the aperture size D and focal distance $Z_F$.

The controller 260 may thus form the optimum scan line transmit beam width W by determining the aperture size D and focal distance $Z_F$ such that a sum of multiple transmit beam widths $W_1$ to $W_n$ has a minimum value.

For example, referring to FIG. 17, the width W of transmit beam to be transmitted along the scan line is determined to be a sum of transmit beam widths $W_A$ and $W_B$ at respective blood flow locations, and the controller 260 may determine the focal distance and aperture size such that the transmit beam width W for the scan line is minimum.

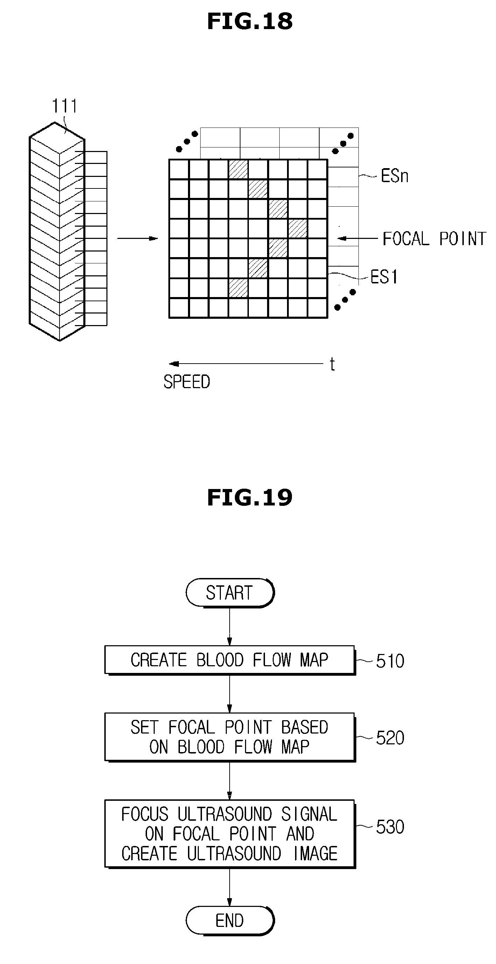

FIG. 18 is a schematic diagram for explaining a synthetic aperture beamforming scheme.

Although the beamformer 210 has been described as a means to focus ultrasound signals on a focal point in a dynamic receive beamforming scheme, it is not limited thereto. The beamformer 210 may employ many different beamforming schemes for focusing ultrasound signals on a focal point or in a focal region determined by the controller 260.

For example, the beamformer 210 may perform beamforming in a synthetic aperture beamforming scheme that performs beamforming without forming a transmit beam, unlike the aforementioned embodiment of the beamformer 210.

The synthetic aperture beamforming scheme may eliminate the need of the beamformer 210 to form a transmit beam. In the synthetic aperture beamforming scheme, the beamformer 210 may control at least one transducer element to transmit an ultrasound. The ultrasound transmitted from the transducer element may be a plane wave.

The beamformer 210 may sequentially store echo signals reflected and returning from the subject ob. The beamformer 210 may only store echo signals output from selected ones of the multiple transducer elements. For example, the beamformer 210 may store a first set of echo signals ES1 generated by sequentially recording the echo signals output from the transducer array 111.

The beamformer 210 may consecutively store sets of echo signals ES1 to ESn while changing the transducer elements that transmit or receive ultrasounds. After completion of storing the multiple sets of echo signals ES1 to ESn, the beamformer 210 may draw echo signals out of the sets of echo signals ES1 to ESn and perform beamforming on them, such that the ultrasound signals are focused on the focal point of each scan line.

For example, the beamformer 210 may draw out first input echo signals for the transducer elements adjacent to the focal point while drawing out last input echo signals for the transducer elements far from the focal point, and perform beamforming on them, as shown in FIG. 18.

FIG. 19 is a flowchart illustrating a method for controlling an ultrasonic diagnostic apparatus, according to an embodiment of the present disclosure.

Referring to FIG. 19, the ultrasonic diagnostic apparatus 1 creates a blood flow map, in operation 510. Specifically, the ultrasonic diagnostic apparatus 1 may collect blood flow information by detecting blood flows in the subject ob, and create a blood flow map based on the collected blood flow information. There are no limitations on collecting the blood flow information. For example, the ultrasonic diagnostic apparatus 1 may detect location and distribution of blood flow in a space domain method, a spectral domain method, an image comparison method, or the like. The blood flow map may include information about multiple blood flow locations and distributions generated along the scan line to create an ultrasound image.

The ultrasonic diagnostic apparatus 1 sets a focal point based on the blood flow map, in operation 520. The focal point is set at a blood flow location that appears in the blood flow map. As the blood flow locations are different for the respective scan lines, different focal points may be set for the respective scan lines. Furthermore, if there are multiple blood flows in a single scan line, the ultrasonic diagnostic apparatus 1 may set a focal point for each blood flow location.

The ultrasonic diagnostic apparatus 1 focuses ultrasound signals on the focal point and create an ultrasound image, in operation 530. There are no limitations on beamforming schemes for focusing ultrasound signals.

For example, the ultrasonic diagnostic apparatus 1 may generate a transmit beam such that ultrasound signals are focused on the focal point, irradiate the transmit beam to the subject ob, and generate respective scan lines for the ultrasonic image by focusing echo signals reflected from the subject ob. The ultrasonic diagnostic apparatus 1 may create an ultrasound image by repeating transmission and reception of ultrasounds while alternating the scan lines.

Once multiple focal points are set for the same scan line as described above, the ultrasonic diagnostic apparatus 1 may generate a transmit beam for each focal point and irradiate the transmit beam to the subject ob. Accordingly, with the multiple focal points in the same scan line, a transmit beam may be irradiated on each focal point. As such, through multiple irradiation of transmit beams, an ultrasound image with blood flows clearly shown may be provided.

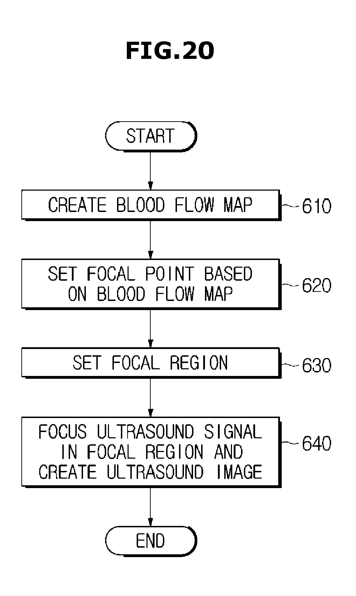

FIG. 20 is a flowchart illustrating a method for controlling an ultrasonic diagnostic apparatus, according to another embodiment of the present disclosure.

Referring to FIG. 20, the ultrasonic diagnostic apparatus 1 creates a blood flow map in operation 610, and sets focal points based on the blood flow map in operation 620.

The ultrasonic diagnostic apparatus 1 may set a focal region, in operation 630. The ultrasonic diagnostic apparatus 1 may set the focal region in which ultrasound signals are to be focused, based on multiple focal points. The focal region may be set to have a size that covers all the multiple focal points.

The multiple focal points used for setting the focal region may be located in the same scan line, but are not limited thereto. For example, the focal region may be set based on multiple focal points located in multiple scan lines that are adjacent to each other.

The ultrasonic diagnostic apparatus 1 focuses ultrasound signals in the focal region and create an ultrasound image, in operation 640. The focal region may be set differently for each scan line.

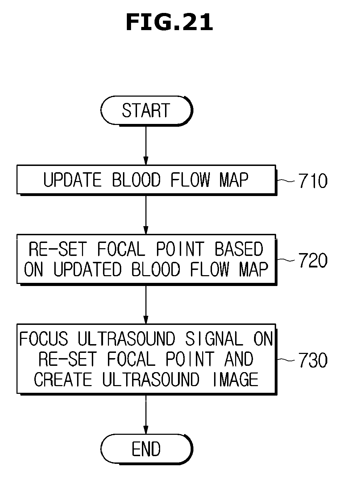

FIG. 21 is a flowchart illustrating a method for controlling an ultrasonic diagnostic apparatus, according to another embodiment of the present disclosure. Beamforming may be dynamically controlled based on the blood flow map obtained in real time.

As shown in FIG. 21, the ultrasonic diagnostic apparatus 1 updates the blood flow map, in operation 710. The ultrasonic diagnostic apparatus 1 may update the blood flow map in real time based on synthesized signals obtained by focusing ultrasound signals on the focal points set based on the blood flow map. Alternatively, the ultrasonic diagnostic apparatus 1 may create an ultrasound image based on synthesized signals obtained by focusing ultrasound signals on the focal points set based on the blood flow map, and update the blood flow map in real time based on the ultrasound image.

The ultrasonic diagnostic apparatus 1 re-sets a focal point based on the updated blood flow map, in operation 720. The focal point may be re-set at a blood flow location of the subject ob that appears on the updated blood flow map. This re-setting procedure may be performed for each scan line.

The ultrasonic diagnostic apparatus 1 obtains an ultrasound image by beamforming such that ultrasound signals are to be focused on the re-set focal point, in operation 730. In other words, the ultrasonic diagnostic apparatus 1 obtains an ultrasonic image by focusing ultrasound signals on the blood flow location in real time. This dynamic beamforming control may enable a clearer ultrasound image to be provided for the user.

The ultrasonic diagnostic apparatus and method for controlling the same in accordance with the aforementioned embodiments of the present disclosure may provide the user with an ultrasound image with a blood flow clearly seen.

Several embodiments have been described above, but a person of ordinary skill in the art will understand and appreciate that various modifications can be made without departing the scope of the present disclosure. Thus, it will be apparent to those ordinary skilled in the art that the true scope of technical protection is only defined by the following claims.

**Claims**

1. An ultrasonic diagnostic apparatus comprising:

   an ultrasonic probe for transmitting or receiving ultrasound signals;
   a blood flow detector for collecting blood flow information by detecting blood flows in a subject;
   a controller for setting at least one focal point in the blood flow of the subject based on the blood flow information; and
   a beamformer for focusing the ultrasound signals on the at least one focal point.

2. The ultrasonic diagnostic apparatus of claim 1,
   wherein the blood flow information includes at least one of location of blood flow and distribution of blood flow.

3. The ultrasonic diagnostic apparatus of claim 2,
   wherein the blood flow detector uses the location and distribution of blood flow detected for each scan line to create a blood flow map.

4. The ultrasonic diagnostic apparatus of claim 1,
   wherein the controller sets the at least one focal point for each scan line.

5. The ultrasonic diagnostic apparatus of claim 4,
   wherein the beamformer forms a transmit beam such that the ultrasound signals are focused on the at least one focal point for each scan line.

6. The ultrasonic diagnostic apparatus of claim 5,
   wherein the beamformer forms a transmit beam per focal point, if there are multiple focal points in the same scan line.

7. The ultrasonic diagnostic apparatus of claim 1,
   wherein the controller determines a focal region for each scan line, in which the ultrasound signals are to be focused, based on the at least one focal point.

8. The ultrasonic diagnostic apparatus of claim 7,
   wherein the beamformer forms a transmit beam such that the ultrasound signals are focused in the focal region determined for each scan line.

9. The ultrasonic diagnostic apparatus of claim 10,
   wherein the controller determines a width of the transmit beam to be irradiated to the scan line.

10. The ultrasonic diagnostic apparatus of claim 9,
    wherein the controller determines a focusing location and focusing range of the transmit beam such that the width of the transmit beam becomes minimum.

11. The ultrasonic diagnostic apparatus of claim 1,
    wherein the beamformer focuses ultrasound signals received from the subject in a synthetic aperture beamforming scheme.

12. The ultrasonic imaging apparatus of claim 11,
    further comprising: a memory for storing ultrasound signals received from the subject, wherein ultrasound signals stored in the memory is drawn out and subject to transmit beamforming, such that ultrasound signals received from the subject are focused on the at least one focal point.

13. The ultrasonic diagnostic apparatus of claim 3,
    wherein the blood flow detector detects the blood flow location for each scan line of the ultrasound signals, and creates the blood flow map for each scan line.

14. The ultrasonic diagnostic apparatus of claim 3,
    wherein the blood flow detector detects a blood flow location that changes according to a synthesized signal generated based on the at least one focal point, and updates the blood flow map based on the changed blood flow location.

**15.** A method for controlling an ultrasonic diagnostic apparatus, the method comprising:

performing detection of blood flow by collecting blood flow information by detecting blood flows in a subject;
setting at least one focal point in the blood flow of the subject based on the blood flow information; and
performing beamforming by focusing the ultrasound signals on the at least one focal point._

# FIG. 1

# FIG.2

# FIG.3

ELEVATIONAL DIRECTION(z)

LATERAL DIRECTION(y)

AXIAL DIRECTION(x)

111

FINAL SCAN LINE

TRANSDUCER ELEMENT

SCANNING DIRECTION

M'TH SCAN LINE

FIRST SCAN LINE

# FIG.4

210

111

213

212

211

| FIRST DELAYER | ← | SIGNAL GENERATOR |

216

217

215

218

| PRE-PROCESSOR | → | SECOND DELAYER | → | SYNTHESIZER |

FIG. 5

FIG. 6

EP 3 050 514 A1

## FIG.7

D2
D1
D3

a'th
SCAN LINE

b'th
SCAN LINE

## FIG.8

220

221 | 222 | 223 | 224

| FILTERING UNIT | FLOW DETECTOR | LOCATION SEARCHING UNIT | BLOOD FLOW MANAGER |

# FIG.9

MAGNITUDE

CLUTTER FILTER

CLUTTER

BLOOD FLOW

SPEED

# FIG.10

BLOOD FLOW
INTENSITY

THRESHOLD

DEPTH

D1

# FIG.11

# FIG.12

# FIG.13

Dc1
Dd1
Pc
Pd
c'th
SCAN LINE
d'th
SCAN LINE

# FIG.14

Dc2
Pc
Pd
Dd2
c'th
SCAN LINE
d'th
SCAN LINE

# FIG.15A

# FIG.15B

# FIG.16

# FIG.17

# FIG.18

ESn

FOCAL POINT

ES1

SPEED       t

# FIG.19

START

CREATE BLOOD FLOW MAP — 510

SET FOCAL POINT BASED ON BLOOD FLOW MAP — 520

FOCUS ULTRASOUND SIGNAL ON FOCAL POINT AND CREATE ULTRASOUND IMAGE — 530

END

# FIG.20

```
          ┌─────────┐
          │  START  │
          └─────────┘
               │
               ▼
   ┌──────────────────────┐
   │ CREATE BLOOD FLOW MAP │──610
   └──────────────────────┘
               │
               ▼
   ┌──────────────────────┐
   │  SET FOCAL POINT BASED │
   │   ON BLOOD FLOW MAP    │──620
   └──────────────────────┘
               │
               ▼
   ┌──────────────────────┐
   │    SET FOCAL REGION   │──630
   └──────────────────────┘
               │
               ▼
   ┌──────────────────────┐
   │ FOCUS ULTRASOUND SIGNAL│
   │   IN FOCAL REGION AND  │──640
   │ CREATE ULTRASOUND IMAGE │
   └──────────────────────┘
               │
               ▼
          ┌─────────┐
          │   END   │
          └─────────┘
```

# FIG.21

```
                    ┌─────────────┐
                    │    START    │
                    └──────┬──────┘
                           │
                           ▼
        ┌─────────────────────────────────┐
        │     UPDATE  BLOOD  FLOW  MAP     │──~710
        └─────────────────┬───────────────┘
                           │
                           ▼
        ┌─────────────────────────────────┐
        │   RE-SET  FOCAL  POINT  BASED    │
        │   ON  UPDATED  BLOOD  FLOW  MAP  │──~720
        └─────────────────┬───────────────┘
                           │
                           ▼
        ┌─────────────────────────────────┐
        │  FOCUS  ULTRASOUND  SIGNAL  ON   │
        │    RE-SET  FOCAL  POINT  AND     │──~730
        │   CREATE  ULTRASOUND  IMAGE      │
        └─────────────────┬───────────────┘
                           │
                           ▼
                    ┌─────────────┐
                    │     END     │
                    └─────────────┘
```

Europäisches
Patentamt

European
Patent Office

Office européen
des brevets

# EUROPEAN SEARCH REPORT

**Application Number**

EP 15 17 2029

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 158 845 A1 (MEDISON CO LTD [KR]) 3 March 2010 (2010-03-03) * abstract * * paragraph [0006] * * paragraph [0012] - paragraph [0017] * * figures 3-6 * | 1-15 | INV. A61B8/06 A61B8/08 A61B8/00 |
| A | US 5 908 391 A (MUZILLA DAVID JOHN [US] ET AL) 1 June 1999 (1999-06-01) * abstract * * column 1, line 16 - line 35 * * column 2, line 3 - column 3, line 4 * * column 4, line 8 - line 26 * * column 5, line 24 - column 7, line 24 * * figures 3-5 * | 1-15 | |
| A | EP 1 568 323 A1 (TOSHIBA KK [JP]; TOSHIBA MEDICAL SYS CORP [JP]) 31 August 2005 (2005-08-31) * abstract * * paragraph [0033] - paragraph [0039] * * figures 5A-5D * | 1-15 | |

**TECHNICAL FIELDS SEARCHED (IPC)**

A61B

The present search report has been drawn up for all claims

| Place of search | Date of completion of the search | Examiner |
|---|---|---|
| Munich | 21 June 2016 | Montes, Pau |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 17 2029

This annex lists the patent family members relating to the patent documents cited in  the above-mentioned European search report.
The members are as contained in the European Patent Office EDP file on
The European Patent Office is in no way liable for these particulars which are merely given for the purpose of information.

21-06-2016

| Patent document cited in search report | | Publication date | Patent family member(s) | | Publication date |
|---|---|---|---|---|---|
| EP 2158845 | A1 | 03-03-2010 | EP | 2158845 A1 | 03-03-2010 |
| | | | JP | 2010051798 A | 11-03-2010 |
| | | | KR | 20100025102 A | 09-03-2010 |
| | | | US | 2010056923 A1 | 04-03-2010 |
| US 5908391 | A | 01-06-1999 | CN | 1208599 A | 24-02-1999 |
| | | | DE | 19819893 A1 | 12-11-1998 |
| | | | IL | 124166 A | 29-06-2000 |
| | | | IT | MI980931 A1 | 02-11-1999 |
| | | | JP | 4324256 B2 | 02-09-2009 |
| | | | JP | H1128209 A | 02-02-1999 |
| | | | NO | 982060 A | 09-11-1998 |
| | | | US | 5908391 A | 01-06-1999 |
| EP 1568323 | A1 | 31-08-2005 | CN | 1660016 A | 31-08-2005 |
| | | | EP | 1568323 A1 | 31-08-2005 |
| | | | JP | 4521204 B2 | 11-08-2010 |
| | | | JP | 2005237738 A | 08-09-2005 |
| | | | US | 2005203406 A1 | 15-09-2005 |

EPO FORM P0459

For more details about this annex : see Official Journal of the European Patent Office, No. 12/82